# EUROPEAN PATENT APPLICATION

(11) **EP 1 875 897 A1**
(43) Date of publication of application: **09.01.2008**
(21) Application number: 07252697.3
(22) Date of filing: 05.07.2007
(51) Int. Cl.: A61K 9/48, A61K 36/48, A61K 8/00

(54) **Preparations for reviving, stimulating and enhancing hair growth**

(30) Priority: 08.07.2006 GB 0613650
(71) Applicant: Arcon Scandinavia APS, 6700 Esbjerg (DK)
(72) Inventor: Marshall, Burkhard F.K., 6700 Esbjerg (DK)
(74) Representative: Brown, Michael Stanley

(57) **Abstract**

A composition for promoting hair growth includes fenugreek extract and soy extract in capsule form.

## Description

### Field of the Invention

This invention relates to preparations for reviving, stimulating and enhancing hair growth.

In British Patent Specification No. 2 359 021 there is described a composition for promoting hair growth that contains fenugreek extract in the form of a lipospray. The composition is applied sub-lingually and the fenugreek extract is absorbed trans-lingually into the bloodstream. The lipospray contains glycerine and hydroxylated lethicin to aid absorption.

It is an object of the present invention to provide an improved composition for promoting hair growth.

Another object of the present invention is the provision of a composition for promoting hair growth that contains fenugreek extract and is available in capsule form.

### Summary of the Invention

According to the present invention there is provided a composition for promoting hair growth that includes fenugreek extract and soy extract in capsule form.

The ratio of the weight of fenugreek extract within the capsule to the weight of soy extract is preferably within the range of from 5:1 to 10:1. The preferred ratio is between 7:1 and 7.5:1.

The fenugreek extract and the soy extract are preferably mixed with an excipient. The excipient may comprise rice powder and silicon dioxide. The excipient may also contain magnesium stearate.

The amount by weight of fenugreek extract in the capsule is preferably within the range of from 200 to 400 milligrams, the preferred weight being approximately 300 milligrams, for example 290 milligrams. The fenugreek extract may be diluted prior to admixture with the other ingredients. A possible dilution rate is 4:1.

The formulation may also include nettle root extract or some other DHT (dihydrotetosterone) inhibitor.

### Description of the Preferred Embodiments

In one embodiment of the invention, a composition in the form of a capsule comprises the following:-
Active ingredients:
   Fenugreek extract - 290 mg.
   Soy extract - 40 mg.
   Riboflavin - 1.5 mg.
   Niacin - 10 mg.
   Vitamin B6 - 2 mg. And
   Pantothenic Acid (as Calcium Pantothenate - 9 mg.
Excipients:
   Pasteurised white rice powder - 74.4 mg.,
   Silicon dioxide - 5.3 mg., and
   Magnesium stearate - 5.3 mg.
Capsule material:
   Capsules Size 0 - Vegetable clear hard shell - 96 mg.

Tests have been carried out on both men and women and all the test subjects took one capsule a day containing the ingredients referred to above for a period of six months.

After taking the capsules for a period of six months, all the test subjects said that their hair felt stronger. For men taking the above capsules, 16.3% had a slight increase in the measured amount of hair, 65.1% had a moderate increase in the measured amount of hair, and 18.6% had a clear increase in the measured amount of hair. There were less women test subjects and the results for these showed that 40% had a slight increase in the measured amount of hair, 40% a moderate increase in the measured amount of hair and 20% a clear increase in the measured amount of hair.

The test subjects were divided into three age groups to assess the variation of the effect of the capsules among the three age groups. For the age group of 20 to 35 years, 11.8% had a slight increase in the amount of hair, 70.6% had a moderate increase in the amount of hair, and 17.6% a clear increase in the measured amount of hair.

For the age group of 36 to 50 years, the corresponding figures were 13.3%, 66.7% and 20%, and for the age group 51 to 70 years, the corresponding figures were 31.2%, 50.0% and 18.8%.

Questions were asked concerning the length of time for which the subjects had suffered hair loss and these were again divided into three groups, i.e. those who had suffered hair loss for up to 6 years, those who had suffered hair loss for between 7 and 15 years and those who had suffered hair loss for more than 16 years. No significant differences were noted between the three groups.

The formulation may also include folic acid and/or nettle root extract or some other DHT (dihydrotetosterone) inhibitor.

## Claims

1. A composition for promoting hair growth that includes fenugreek extract and soy extract in capsule form.

2. A composition as claimed in Claim 1, in which the ratio of the weight of fenugreek extract within the or each capsule to the weight of soy extract is within the range of from 5:1 to 10:1.

3. A composition as claimed in Claim 2, in which the ratio of the weight of fenugreek extract to the weight of soy extract is between 7:1 and 7.5:1.

4. A composition as claimed in any one of the preceding claims, in which the fenugreek extract and the soy extract are mixed with an excipient.

5. A composition as claimed in Claim 4, in which the excipient comprises rice powder and silicon dioxide.

6. A composition as claimed in Claim 5, in which the excipient also contains magnesium stearate.

7. A composition as claimed in any one of the preceding claims, in which the amount by weight of fenugreek extract in the or each capsule is within the range of from 200 to 400 milligrams.

8. A composition as claimed in Claim 7, in which the amount by weight of fenugreek extract is approximately 300 milligrams.

9. A composition as claimed in any one of the preceding claims, which also includes a dihydrotetosterone inhibitor.

10. A composition as claimed in Claim 9, in which the dihydrotesterone inhibitor is nettle root extract.
